# NEW EUROPEAN PATENT SPECIFICATION

(11) **EP 3 044 357 B3**
(45) Date of publication of this specification: **14.07.2021**
(45) Mention of the grant of the patent: 07.11.2018
(21) Application number: 14843549.8
(22) Date of filing: 12.09.2014
(51) Int. Cl.: D03D 15/08, D01F 6/70, D01F 8/00, A61F 13/49, A61F 13/15, D01F 1/10, D01F 6/72, D01F 6/94, D01F 8/16

(54) **SPANDEX FIBERS FOR ENHANCED BONDING**
SPANDEXFASERN ZUR VERBESSERTEN VERKLEBUNG
FIBRES SPANDEX À LIAGE AMÉLIORÉ

(30) Priority: 13.09.2013 US 201361877609 P
(43) Date of publication of application: 20.07.2016
(73) Proprietor: The LYCRA Company UK Limited, Manchester M2 3DE (GB)
(72) Inventor: BIVIGOU-KOUMBA, Achille M., Greensboro, NC 27401 (US); LIU, Hong, Waynesboro, Virginia 22980 (US); BING-WO, Ronald D., Waynesboro, Virginia 22980 (US); SMITH, Steven W., Waynesboro, Virginia 22980 (US)
(74) Representative: Carpmaels & Ransford LLP
(86) International application number: PCT/US2014/055519
(87) International publication number: WO 2015/038977

(56) References cited:
- WO-A1-2007/070101
- WO-A2-2010/045155
- WO-A2-2011/149734
- JP-A- H0 214 056
- JP-A- H04 316 609
- US-A- 4 251 642
- US-A1- 2004 106 733
- US-A1- 2004 106 733
- US-A1- 2007 135 008

## Description

### Background of the Invention

### Field of the Invention

The present invention relates to the formation of elastic fibers (either bicomponent such as spandex sheath-core fibers) or a single component (also referred to herein as monocomponent fiber) suitable for bonding to a substrate such as one includes polyolefin fiber. The spandex fibers are produced by dry spinning of polymer blends into a monocomponent or sheath-core bicomponent fiber.

### Description of the Related Art

The global market for disposable diapers has an increased demand for fibers with excellent resilience, high stretchability and high bonding capacity to nonwovens. Because of their outstanding properties, including high elongation and good elastic recovery, spandex fibers continue to gain interest for their use as elastic strands in disposable diapers. In incontinence products such as baby and adult diapers spandex fibers are used in several locations such as in leg bands and/or waistbands to provide an excellent fit of the diapers.

To introduce stretchable regions in disposable diapers, a hot melt adhesive is applied against a stretched elastomeric fiber which is later laminated between two or more layers such as films or nonwovens that may include polypropylene. While hot melt adhesives are applied to create bond sites between elastomeric strands and nonwoven substrates, significant lack of adhesion is still observed between the different components of the diaper: spandex strands, hot melt adhesive and nonwoven fabrics. It is believed that hot melt adhesives are compatible with nonwoven fabrics, while they are chemically incompatible with spandex fibers.

To address this drawback, the most popular method available for bonding spandex fibers to nonwoven fabrics is to overload spandex strands with a hot melt adhesive. Since the cost of hot melt adhesives is high, it is convenient to apply adhesive add-ons lower than 18 gsm (grams of adhesive material per square meter of substrate covered by the adhesive), more preferably equal to or lower than 15 gsm, and most preferably equal to or lower than 12 gsm.

U.S. Patent No. 7,255,820 discloses the use of polystyrene in spandex, but only for the purpose of achieving improved heat set, which is unrelated to the technical problem disclosed herein.

### Summary of the Invention

Blend spinning spandex with an economical alternative polymer additive provides a route to reduce adhesive consumption cost with no change in spandex fibers performance. Of the unique benefits sheath-core fibers arrangement have over the conventional one, is a potential cost savings associated with the use of less of a more expensive polymeric additive to obtain the same desirable characteristics or adding an expensive additive to only the sheath polymer, taking advantage of the lower melting point of a polymer spun in the sheath to promote bonding without a disruption in the morphology of the core component, improving the efficiency of elastic fibers made from a spinnable polymer core sheathed within a polymer blend comprising polymers with poor and good spinnability.

Some embodiments provide monocomponent and/or bicomponent sheath-core spandex fibers with bonding enhancing additives which enhance bonding between spandex fibers to substrates, such as nonwovens. The sheath-core fibers include a higher-melting polyurethaneurea core and a sheath including a low-melting thermoplastic polymer as a bonding enhancing additive.

The reason for using low-melting polymer sheath is to provide a higher bonding strength with adhesives and substrates such as nonwovens or, in the alternative, to reduce the amount of adhesive required for the same bonding strength.

In one embodiment the elastic material is attached to the polyolefin nonwoven substrate when the melt adhesive is sprayed onto the elastic strands at temperatures range 160°C-200°C, which is above the glass transition temperature of the thermoplastic polymer sheath, the latter melts or softens the bonding enhancing additive to form bond sites with hot melt adhesives and nonwoven substrates upon cooling.

The thermoplastic binders include polystyrene. The primary reason for using polystyrene polymers is that they are inherently capable of bonding to hot melt adhesive since these adhesives are mostly made of polystyrene block copolymers. Finally, the low cost of polystyrene polymers is another attractive reason to increases cost saving in disposable diapers applications.

In one embodiment of a sheath-core elastic fiber, the core fibers include 100% of polyurethane such as a higher-melting segmented polyurethane/urea polymer and the sheath includes a polymer blend including about 30% to 99.5% of at least one selected polyurethane/urea polymer and 0.5% to 70% of at least one additive such as an enhanced bonding additive. The elastic monocomponent and bicomponent sheath-core fibers are capable of providing enhanced bonding to substrates such as a polyolefin fiber substrate (including nonwovens) when the former are heated above the corresponding glass transition or melting temperature of the enhanced bonding additive, optionally in the presence of a hot melt adhesive. As an additional benefit, reduced tack of the fiber has been observed which improves over-end take-off tension in dispenses spandex.

### Detailed Description of the Invention

Laminate structures are provided that include an elastic fiber including polyurethane and 0.01% to 30% by weight of at least one additive selected from polystyrene; where the elastic fiber is adhered to one or more layers of a substrate selected from the group consisting of fabric, nonwoven, film, and combinations thereof. The laminate structure may be adhered by any known method, including but not limited to where the elastic fiber is adhered by an adhesive, ultrasonic bonding, thermal bonding or combinations thereof.

The elastic fiber, which can be used in a fabrics or laminate structures may include a topical finish including mineral oil, silicon oil, or combinations thereof. This finish may be present in any suitable amount such as an amount of 0.1% to 2% by weight of the fiber including up to 1% by weight of the fiber. The topical finish has a viscosity of 5 centistokes to 150 centistokes.

Other additives can be included in the fiber as desired. Suitable fiber additives include magnesium stearate, organic stearates, silicon oil, mineral oil, and combinations thereof.

The elastic fiber may include polyurethane and/or polyurethaneurea and 0.01 % to 0.90% (including 0.3% to 0.85% and 0.60% to 0.85%) by weight of at least one additive selected from polystyrene. This fiber may be solution-spun. The fiber may have a homogeneous cross-section (a monocomponent fiber) or have a bicomponent elastic fiber comprising a sheath-core construction; where the core includes polyurethane and said sheath comprises at least one additive selected from polystyrene.

The elastic fiber may also be included in fabric, such as a knit, a woven, or a nonwoven construction. The elastic fiber includes polyurethane and 0.01% to 30% by weight of at least one additive selected from polystyrene.

A method for preparing a laminate structure is provided including:
providing an elastic fiber including polyurethane and 0.01% to 30% (such as 0.1 % to 3.0%) by weight of at least one additive selected from polystyrene;
where the elastic fiber is adhered to one or more layers of a substrate selected from the group consisting of fabric, nonwoven, film, and combinations thereof.

The laminate structure may be adhered by ultrasonic bonding, an adhesive, thermal bonding, and combinations thereof. This laminate article may be included in a disposable hygiene article.

### Polyurethaneurea and Polyurethane Compositions

Polyurethaneurea compositions useful for preparing fiber or long chain synthetic polymers that include at least 85% by weight of a segmented polyurethane. Typically, these include a polymeric glycol or polyol which is reacted with a diisocyanate to form an NCO-terminated prepolymer (a "capped glycol"), which is then dissolved in a suitable solvent, such as dimethylacetamide, dimethylformamide, or N-methylpyrrolidone, and then reacted with a difunctional chain extender. Polyurethanes are formed when the chain extenders are diols (and may be prepared without solvent). Polyurethaneureas, a sub-class of polyurethanes, are formed when the chain extenders are diamines. In the preparation of a polyurethaneurea polymer which can be spun into spandex, the glycols are extended by sequential reaction of the hydroxy end groups with diisocyanates and one or more diamines. In each case, the glycols must undergo chain extension to provide a polymer with the necessary properties, including viscosity. If desired, dibutyltin dilaurate, stannous octoate, mineral acids, tertiary amines such as triethylamine, N,N'-dimethylpiperazine, and the like, and other known catalysts can be used to assist in the capping step.

Suitable polyol components include polyether glycols, polycarbonate glycols, and polyester glycols of number average molecular weight of 600 to 3,500. Mixtures of two or more polyols or copolymers can be included.

Examples of polyether polyols that can be used include those glycols with two or more hydroxy groups, from ring-opening polymerization and/or copolymerization of ethylene oxide, propylene oxide, trimethylene oxide, tetrahydrofuran, and 3-methyltetrahydrofuran, or from condensation polymerization of a polyhydric alcohol, such as a diol or diol mixtures, with less than 12 carbon atoms in each molecule, such as ethylene glycol, 1,3-propanediol, 1,4-butanediol, 1,5-pentanediol 1,6-hexanediol, neopentyl glycol, 3-methyl-1,5-pentanediol, 1,7-heptanediol, 1,8-octanediol, 1,9-nonanediol, 1,10-decanediol and 1,12-dodecanediol. A linear, bifunctional polyether polyol is preferred, and a poly(tetramethylene ether) glycol of molecular weight of 1,700 to 2,100, such as Terathane® 1800 (INVISTA of Wichita, KS) with a functionality of 2, is one example of a specific suitable polyol. Co-polymers can include poly(tetramethylene-co-ethyleneether) glycol.

Examples of polyester polyols that can be used include those ester glycols with two or more hydroxy groups, produced by condensation polymerization of aliphatic polycarboxylic acids and polyols, or their mixtures, of low molecular weights with no more than 12 carbon atoms in each molecule. Examples of suitable polycarboxylic acids are malonic acid, succinic acid, glutaric acid, adipic acid, pimelic acid, suberic acid, azelaic acid, sebacic acid, undecanedicarboxylic acid, and dodecanedicarboxylic acid. Examples of suitable polyols for preparing the polyester polyols are ethylene glycol, 1,3-propanediol, 1,4-butanediol, 1,5-pentanediol 1,6-hexanediol, neopentyl glycol, 3-methyl-1,5-pentanediol, 1,7-heptanediol, 1,8-octanediol, 1,9-nonanediol, 1,10-decanediol and 1,12-dodecanediol. A linear bifunctional polyester polyol with a melting temperature of 5°C to 50°C is an example of a specific polyester polyol.

Examples of polycarbonate polyols that can be used include those carbonate glycols with two or more hydroxy groups, produced by condensation polymerization of phosgene, chloroformic acid ester, dialkyl carbonate or diallyl carbonate and aliphatic polyols, or their mixtures, of low molecular weights with no more than 12 carbon atoms in each molecule. Examples of suitable polyols for preparing the polycarbonate polyols are diethylene glycol, 1,3-propanediol, 1,4-butanediol, 1,5-pentanediol, 1,6-hexanediol, neopentyl glycol, 3-methyl-1,5-pentanediol, 1,7-heptanediol, 1,8-octanediol, 1,9-nonanediol, 1,10-decanediol and 1,12-dodecanediol. A linear, bifunctional polycarbonate polyol with a melting temperature of 5°C to 50°C is an example of a specific polycarbonate polyol.

The diisocyanate component can also include a single diisocyanate or a mixture of different diisocyanate including an isomer mixture of diphenylmethane diisocyanate (MDI) containing 4,4'-methylene bis(phenyl isocyanate) and 2,4'- methylene bis(phenyl isocyanate). Any suitable aromatic or aliphatic diisocyanate can be included. Examples of diisocyanates that can be used include, but are not limited to, 1-isocyanato-4-[(4-isocyanatophenyl)methyl]benzene, 1-isocyanato-2-[(4-cyanatophenyl)methyl]benzene, bis(4-isocyanatocyclohexyl)methane, 5-isocyanato-1-(isocyanatomethyl)-1,3,3-trimethylcyclohexane, 1,3-diisocyanato-4-methylbenzene, 2,2'-toluenediisocyanate, 2,4'-toluenediisocyanate, and mixtures thereof. Examples of specific polyisocyanate components include Mondur® ML (Bayer), Lupranate® MI (BASF), and Isonate® 50 O,P' (Dow Chemical), and combinations thereof.

A chain extender may be either water or a diamine chain extender for a polyurethaneurea. Combinations of different chain extenders may be included depending on the desired properties of the polyurethaneurea and the resulting fiber. Examples of suitable diamine chain extenders include: hydrazine; 1,2-ethylenediamine; 1,4-butanediamine; 1,2-butanediamine; 1,3-butanediamine; 1,3-diamino-2,2-dimethylbutane; 1,6-hexamethylenediamine; 1,12-dodecanediamine; 1,2-propanediamine; 1,3-propanediamine; 2-methyl-1,5-pentanediamine; 1-amino-3,3,5-trimethyl-5-aminomethylcyclohexane; 2,4-diamino-1-methylcyclohexane; N-methylamino-bis(3-propylamine); 1,2-cyclohexanediamine; 1,4-cyclohexanediamine; 4,4'-methylene-bis(cyclohexylamine); isophorone diamine; 2,2-dimethyl-1,3-propanediamine; *meta-*tetramethylxylenediamine; 1,3-diamino-4-methylcyclohexane; 1,3-cyclohexane-diamine; 1,1-methylene-bis(4,4'-diaminohexane); 3-aminomethyl-3,5,5-trimethylcyclohexane; 1,3-pentanediamine (1,3-diaminopentane); m-xylylene diamine; and Jeffamine® (Texaco).

When a polyurethane is desired, the chain extender is a diol. Examples of such diols that may be used include, but are not limited to, ethylene glycol, 1,3-propanediol, 1,2-propylene glycol, 3-methyl-1,5-pentanediol, 2,2-dimethyl-1,3-trimethylene diol, 2,2,4-trimethyl-1,5-pentanediol, 2-methyl-2-ethyl-1,3-propanediol, 1,4-bis(hydroxyethoxy)benzene, and 1,4-butanediol and mixtures thereof.

A blocking agent which is a monofunctional alcohol or a monofunctional dialkylamine may optionally be included to control the molecular weight of the polymer. Blends of one or more monofunctional alcohols with one or more dialkylamine may also be included.

Examples of monofunctional alcohols useful with the present invention include at least one member selected from the group consisting of aliphatic and cycloaliphatic primary and secondary alcohols with 1 to 18 carbons, phenol, substituted phenols, ethoxylated alkyl phenols and ethoxylated fatty alcohols with molecular weight less than 750, including molecular weight less than 500, hydroxyamines, hydroxymethyl and hydroxyethyl substituted tertiary amines, hydroxymethyl and hydroxyethyl substituted heterocyclic compounds, and combinations thereof, including furfuryl alcohol, tetrahydrofurfuryl alcohol, N-(2-hydroxyethyl)succinimide, 4-(2-hydroxyethyl)morpholine, methanol, ethanol, butanol, neopentyl alcohol, hexanol, cyclohexanol, cyclohexanemethanol, benzyl alcohol, octanol, octadecanol, N,N-diethylhydroxylamine, 2-(diethylamino)ethanol, 2-dimethylaminoethanol, and 4-piperidineethanol, and combinations thereof.

Examples of suitable mono-functional dialkylamine blocking agents include: N,N-diethylamine, N-ethyl-N-propylamine, N,N-diisopropylamine, N-*tert-*butyl-N-methylamine, N-*tert*-butyl-N-benzylamine, N, N-dicyclohexylamine, N-ethyl-N-isopropylamine, N-*tert*-butyl-N-isopropylamine, N-isopropyl-N-cyclohexylamine, N-ethyl-N-cyclohexylamine, N,N-diethanolamine, and 2,2,6,6-tetramethylpiperidine.

### Bonding Enhancing Additives

The bonding enhancing additives are selected from polystyrene..

The bonding enhancing additives may be of any suitable molecular weight such as number average molecular weight of 300 to 500,000. A higher number average molecular weight may be 50,000-500,000 or 150,000 to 300,000. A lower number average molecular weight may be less than 50,000. Suitable ranges for the lower number average molecular weight hydrocarbon resin may be 300 to 45,000 or 1000 to 40,000.

### Additives

Classes of additives that may be optionally included in polyurethaneurea compositions are listed below. An exemplary and non-limiting list is included. However, additional additives are well-known in the art. Examples include: anti-oxidants, UV stabilizers, colorants, pigments, crosslinking agents, phase change materials (paraffin wax), antimicrobials, minerals *(i.e.,* copper), microencapsulated additives *(i.e.,* aloe vera, vitamin E gel, aloe vera, sea kelp, nicotine, caffeine, scents or aromas), nanoparticles *(i.e.,* silica or carbon), calcium carbonate, flame retardants, antitack additives, chlorine degradation resistant additives, vitamins, medicines, fragrances, electrically conductive additives, dyeability and/or dye-assist agents (such as quaternary ammonium salts). Other additives which may be added to the polyurethaneurea compositions include adhesion promoters, anti-static agents, anti-creep agents, optical brighteners, coalescing agents, electroconductive additives, luminescent additives, lubricants, organic and inorganic fillers, preservatives, texturizing agents, thermochromic additives, insect repellants, and wetting agents, stabilizers (hindered phenols, zinc oxide, hindered amine), slip agents (silicone oil) and combinations thereof.

The additive may provide one or more beneficial properties including: dyeability, hydrophobicity (i.e., polytetrafluoroethylene (PTFE)), hydrophilicity (i.e., cellulose), friction control, chlorine resistance, degradation resistance (i.e., antioxidants), adhesiveness and/or fusibility (i.e., adhesives and adhesion promoters), flame retardance, antimicrobial behavior (silver, copper, ammonium salt), barrier, electrical conductivity (carbon black), tensile properties, color, luminescence, recyclability, biodegradability, fragrance, tack control (i.e., metal stearates), tactile properties, set-ability, thermal regulation (i.e., phase change materials), nutriceutical, delustrant such as titanium dioxide, stabilizers such as hydrotalcite, a mixture of huntite and hydromagnesite, UV screeners, and combinations thereof.

### Process of Making Fibers

The fiber of some embodiments is produced by solution spinning (either wet-spinning or dry spinning) of the polyurethane-urea polymer from a solution with conventional urethane polymer solvents (e.g., DMAc). The polyurethaneurea polymer solutions may include any of the compositions or additives described above. The polymer is prepared by reacting an organic diisocyanate with appropriate glycol, at a mole ratio of diisocyanate to glycol in the range of 1.6 to 2.3, preferably 1.8 to 2.0, to produce a "capped glycol". The capped glycol is then reacted with a mixture of diamine chain extenders. In the resultant polymer, the soft segments are the polyether/urethane parts of the polymer chain. These soft segments exhibit melting temperatures of lower than 60°C. The hard segments are the polyurethane/urea parts of the polymer chains; these have melting temperatures of higher than 200°C. The hard segments amount to 5.5 to 9%, preferably 6 to 7.5%, of the total weight of the polymer.

In one embodiment of preparing fibers, the polymer solutions containing 30-40% polymer solids are metered through desired arrangement of distribution plates and orifices to form filaments. Distribution plates are arranged to combine polymer streams in a one of concentric sheath-core, eccentric sheath-core, and side-by-side arrangement followed by extrusion thru a common capillary. Extruded filaments are dried by introduction of hot, inert gas at 300°C-400°C and a gas:polymer mass ratio of at least 10:1 and drawn at a speed of at least 400 meters per minute (preferably at least 600 m/min) and then wound up at a speed of at least 500 meters per minute (preferably at least 750 m/min). All examples given below were made with 80°C extrusion temperature in to a hot inert gas atmosphere at a take-up speed of 762 m/min. Standard process conditions are well-known in the art.

The additive which enhances bonding (polystyrene) may be added to the polymer solution for the core, the sheath only, or for a monocomponent (not bicomponent) fiber. For example, in a sheath-core bicomponent fiber, the additive may be present only in the sheath. When the additive is only in the sheath, it may be present in an amount of 0.5% to 70% by weight of the sheath.

Yarns formed from elastic fibers made in accordance with the present invention generally have a tenacity at break of at least 0.6 cN/dtex, a break elongation of at least 400%, an unload modulus at 300% elongation of at least 27 mg/dtex.

Strength and elastic properties of the spandex were measured in accordance with the general method of ASTM D 2731-72. For the examples reported in Tables below, spandex filaments having a 5 cm gauge length were cycled between 0% and 300% elongation at a constant elongation rate of 50 cm per minute. Modulus was determined as the force at 100% (M100) and 200% (M200) elongation on the first cycle and is reported in grams. Unload modulus (U200) was determined at 200% elongation on the fifth cycle and is reported in the Tables in grams.

Percent elongation at break and force at break was measured on the sixth extension cycle.

Percent set was determined as the elongation remaining between the fifth and sixth cycles as indicated by the point at which the fifth unload curve returned to substantially zero stress. Percent set was measured 30 seconds after the samples had been subjected to five 0-300% elongation/relaxation cycles. The percent set was then calculated as% Set=100(Lf-Lo)/Lo, where Lo and Lf are the filament (yarn) length, when held straight without tension, before (Lo) and after (Lf) the five elongation/relaxation cycles.

The features and advantages of the present invention are more fully shown by the following examples which are provided for purposes of illustration, and are not to be construed as limiting the invention in any way. Laminate structures not in the scope of the claims are provided as reference structures only.

### EXAMPLES

Representative embodiments of the present invention will be described with reference to the following examples that illustrate the principle and practice of the present invention. In these examples, Polyurethane/urea prepared according to a conventional method is a linear polymer (commercially available from Invista, S. a. r. L., of Wichita, KS and Wilmington, DE);
St stands for polystyrene;
St1 stands for lower number average molecular weight polystyrene;
BM stands for poly(butyl methacrylate);
IBM stands for poly(isobutyl methacrylate);
MMA stands for poly(methyl methacrylate);
AN stands for polyacrylonitrile;
SMMA stands for copolymer poly(styrene-co-methyl methacrylate);
SAN stands for copolymer poly(styrene-co-acrylonitrile);
PVP stands for polyvinylpyrrolidone
MB2766: thermoplastic acrylic resin
ZeroCreep™ stands for hot melt elastic attachment adhesive.

The compounds listed above are commercially available from the Sigma Aldrich) except for MB2766 and ZeroCreep™ which were provided by Dianal America, Inc and the hot melt adhesive by Bostik.

### Example 1

### Core solution

Segmented polyurethane was completely dissolved in a dimethylacetamide solvent, to obtain a spinning core solution. As such, a mixing ratio by weight of polyurethane to DMAC was 35:65 (w/w).

### Sheath solution

Polystyrene was completely dissolved in a dimethylacetamide solvent, to obtain a polystyrene stock solution, which was then mixed with a segmented polyurethane solution (see core solution). As such, a mixing ratio by weight of polystyrene to polyurethane is listed in Table 1. To assure uniformity, each copolymer sample was thoroughly mixed for 6h before characterization or spinning was started.

Thereafter, the core-sheath solutions were spun into a single thread of 40 and 360 denier yarns with 4 filaments twisted together at a wound-up speed of 930 meters per minute. Prior to entering the spinning core-sheath cell, which was flushed with nitrogen gas of 375°C at a flow rate of 5.5 kg per hour, the polymer solution temperature was controlled at 50°C. The dried yarn was then winding-up into a tube. The as-spun yarn properties of this test item were measured and listed in Table 1.

### Example 2

The same procedures and conditions were used as described in Example 1 except that polystyrene was replaced either by poly(methyl methacrylate) or poly(acrylonitrile) and a mixing ratio by weight (w/w) shown in Table1. The as-spun yarn properties of this test item were measured and listed in Table 1.

### Example 3

Polystyrene was dissolved in a dimethylacetamide solvent, to obtain a polystyrene solution (30%) and the formed polymer solution was spun into a yarns. The as-spun yarn properties of this test item were measured and listed in Table 3a.

### Example 4

Both polystyrene and polyvinylpyrrolidone were separately dissolved in a dimethylacetamide solvent, to obtain a polystyrene solution (30%) and a polyvinylpyrrolidone solution (20%). Thereafter, a polymer blend solutions made from a polystyrene solution (30%) and polyvinylpyrrolidone solution (20%) were spun into a yarns. The as-spun yarn properties of this test item were measured and listed in Table 3.

### Example 5

Lower average number molecular weight polystyrene was dissolved in a dimethylacetamide solvent, to obtain a polystyrene solution (60%) and the formed polymer solution was spun into a yarns. The as-spun yarn properties of this test item were measured and listed in Table 3b

**Table 1. Comparison of sheath-core spandex as-spun yarn properties with control spandex yarn.**

| Sample Number | P1 | P2 | P3 | P4 | P5 | P6 | P7 | P8 | P9 | P10 | P11 | P12 |
|---|---|---|---|---|---|---|---|---|---|---|---|---|
| Sheath Polymer Type | PUU | PUU/St | PUU/St | PUU/St | PUU/St/ PU | PUU/ SMMA | PUU/ SAN | PUU | PUU/St | PUU/ BM | PUU/ IBM | PUU/ MB2766/St |
| Weight percentage of Sheath in fiber (%) | 0 | 5 | 5 | 5 | 5 | 5 | 5 | 0 | 5 | 5 | 5 | 5 |
| Sheath Composition (%) | 0 | 98.25/1. 75 | 90/10 | 70/30 | 40/30/30 | 90/10 | 90/10 | 0 | 80/20 | 80/20 | 80/20 | 80/10/10 |
| Core Polymer Type | PUU | PUU | PUU | PUU | PUU | PUU | PUU | PUU | PUU | PUU | PUU | PUU |
| Thermoplastic polymer in fiber (%) | 0 | 0.09 | 0.5 | 1.5 | 3 | 0.5 | 0.5 | 0 | 1 | 1 | 1 | 1 |
| Denier | 369 | 379 | 338 | 363 | 364 | 368 | 374 | 370 | 368 | 372 | 369 | 369.4 |
| ELO | 500 | 519 | 524 | 520 | 510 | 537 | 537 | 531 | 540 | 530 | 506 | 529 |
| SET | 26.5 | 26.8 | 27 | 29.3 | 28.8 | 28 | 29 | 26.7 | 27.6 | 28.2 | 28.5 | 28.1 |

| | | | | | | | | | | | | |
|---|---|---|---|---|---|---|---|---|---|---|---|---|
| PUU: Control spandex yarn (polyurethaneurea); PU: Polyurethaneurea; St: Polystyrene; SMMA: Poly(styrene-co-methyl methacrylate); SAN: Poly(styrene-co-acrylonitrile); BM: Poly(butyl methacrylate); IBM: Poly(isobutyl methacrylate); MB2766: Acrylic resins. | | | | | | | | | | | | |

Table 1 illustrates the properties of the sheath-core bicomponent spandex fibers suitable to the present invention. While P1 is the control, the regular spandex, samples P2-10 are sheath-core spandex fibers made according to the present invention. It can be seen from the above examples that dry spinning of a solution mixture of a segmented polyurethane with polymeric additive including but not limited to polystyrene, poly(styrene-co-methyl methacrylate); poly(styrene-co-acrylonitrile), poly(butyl methacrylate) and derivatives has no significant negative impact to the yarn properties. The slight deviations seen in the elongation and set data of these fibers were expected and they are attributable to the addition of polymeric additives into the individual spandex yarns.

Overall, the sheath-core bicomponent spandex fibers P2-10 were found to have excellent properties such as high elongation and similar set to the regular spandex, P1.

### Bonding results

After bonding the bicomponent spandex fibers to nonwovens, the samples were tested for bond retention (100-% Creep Retention) according to the method described earlier. In a diaper application, bond performance for spandex fibers is said to be good typically when the bond retention is either more than 60%, preferably more than 70%, more preferably more than 75%, most preferably more than 80% in a specific test described hereinafter when it is done within 2 days after hot melt adhesive has been applied on substrates. These tests are indicative of what level of adhesion and bond retention can be achieved by a hot melt adhesive. Because of the high cost of hot melt adhesives, the adhesives are used in an add-on amount lower than 18 gsm (grams of adhesive material per square meter of substrate covered by the adhesive material), more preferably equal to or lower than 15 gsm, and most preferably equal to or lower than 12 gsm, U.S. Pat. No. 20090264580.

In the present invention, the bond performance of sheath-core spandex fibers is illustrated by the specific examples shown in Table 2a-b.

**Table 2a-b. Comparison in bonding retention of sheath-core spandex as-spun yarn properties with control spandex yarn using 7, 11, and 15.5 gsm of ZeroCreep™ add-ons.**

| a) | | | | | |
|---|---|---|---|---|---|
| Adhesive add-on (gsm) | % Bond retention | Adhesive add-on (gsm) | % Bond retention | Adhesive add-on (gsm) | % Bond retention |
| 7gsm | | 11gsm | | 15.5gsm | |
| P1 | 50.0 | P1 | 65.0 | P1 | 82.0 |
| P2 | 59.0 | P2 | 77.0 | P2 | 85.0 |
| P3 | 67.0 | P3 | 74.0 | P3 | 84.0 |
| P4 | 66.0 | P4 | 78.0 | P4 | 85.0 |
| P5 | 65.0 | P5 | 76.0 | P5 | 85.0 |
| P6 | 57.0 | P6 | 79.0 | P6 | 83.0 |
| P7 | 63.0 | P7 | 77.0 | P7 | 84.0 |

| b) | | | | | |
|---|---|---|---|---|---|
| Adhesive add-on (gsm) | % Bond retention | Adhesive add-on (gsm) | % Bond retention | Adhesive add-on (gsm) | % Bond retention |
| 7gsm | | 11gsm | | 15.5gsm | |
| P8 | 67.0 | P8 | 75.0 | P8 | 86.0 |
| P9 | 74.0 | P9 | 82.0 | P9 | 88.0 |
| P10 | 72.0 | P10 | 80.0 | P10 | 86.0 |
| P11 | 74.0 | P11 | 79.0 | P11 | 88.0 |
| P12 | 75.0 | P12 | 82.0 | P12 | 88.0 |

From Table 2a-b, it is clear that the inventive bicomponent fibers exhibited greater bond retention to nonwoven fabrics than the control spandex fiber.

When the ZeroCreep™ add-on is 7 gsm, the bicomponent spandex fibers P2-7 and P9-12 display good bond performance than the control spandex fibers P1 or P8 (Table 2a-b). It should be noted that fibers with polystyrene and poly(butyl methacrylate) additives display higher bond retention.

At ZeroCreep™ add-on equivalents to 11 gsm, all six bicomponent fibers claimed by the present invention have superior bondability to nonwovens than the control spandex fiber. As seen previously, the bond retention increased more when polystyrene and poly(butyl methacrylate) are used in fibers sheath.

When the ZeroCreep™ add-on is 15.5 gsm, all inventive fibers (P2-7 and P9-12) including the control ones (P1 and P8) exhibited much higher bond retention. Still, the inventive bicomponent fibers display a better creep retention compared to the control fiber.

**Table 3a. Comparison of monocomponent spandex as-spun yarn properties with control spandex yarn.**

| Sample Number | P13 | P14 | P15 |
|---|---|---|---|
| Polymer Type | PUU1 | PUU1/St | PUU1/St/PVP |
| Thermoplastic polymer in fiber (%) | 0 | 0.875 | 98.25/0.875/0.875 |
| Denier | 745.5 | 739.5 | NA |
| ELO | 492.75 | 520.25 | 500.0 |
| TEN | 423.8 | 522.45 | 409.8 |
| TP2 | 81.06 | 83.72 | 97.71 |
| TM2 | 16.55 | 15.96 | 15.07 |
| SET | 25.55 | 27.95 | 28.5 |

| | | | |
|---|---|---|---|
| PUU1: Control spandex yarn (polyurethaneurea); St: Polystyrene; PVP: polyvinylpyrrolidone. | | | |

**Table 3b. Comparison of monocomponent spandex as-spun yarn properties with control spandex yarn.**

| Sample Number | P16 | P17 | P18 |
|---|---|---|---|
| Polymer Type | PUU2/St1 | PUU2/St1 | PUU2 |
| Thermoplastic polymer in fiber (%) | 2.5 | 4.2 | 0 |
| Target Denier | 720 | 720 | 720 |
| ELO | 465 | 477 | 503 |
| TEN | 380 | 410 | 388 |
| TP2 | 84.2 | 84.8 | 85.3 |
| TM2 | 15.7 | 15.4 | 15.3 |
| SET | 27.3 | 28.1 | 26.5 |

| | | | |
|---|---|---|---|
| PUU2: Control spandex yarn (polyurethaneurea); St1: low average number molecular weight polystyrene | | | |

**Table 4. Comparison in bonding retention of monocomponent spandex as-spun yarn properties with control spandex yarn using 7, 11, and 15.5 gsm of ZeroCreep™ add-ons.**

| Adhesive add-on (gsm) | % Bond retention | Adhesive add-on (gsm) | % Bond retention | Adhesive add-on (gsm) | % Bond retention |
|---|---|---|---|---|---|
| 7gsm | | 11gsm | | 15.5gsm | |
| P13 | 53.0 | P13 | 80.0 | P13 | 87.0 |
| P14 | 63.0 | P14 | 79.0 | P14 | 87.0 |
| P15 | 58.0 | P15 | 78.0 | P15 | 84.0 |

**Table 5. Comparison in bonding retention of monocomponent spandex as-spun yarn properties with control spandex yarn using 15 and 20 mg/m/strand of Technomelt® DM 800B mady be Henkel.**

| Adhesive add-on | % Bond retention | Adhesive add-on | % Bond retention |
|---|---|---|---|
| 15 mg/m/strand | | 20 mg/m/strand | |
| P16 | 82.3 | P16 | 89.2 |
| P17 | 83.5 | P17 | 88.7 |
| P18 | 77.6 | P18 | 87.7 |

Tables 4 and 5 show the inventive fibers exhibit better bond retention than the control.

While there have been described what are presently believed to be the preferred embodiments of the invention, those skilled in the art will realize that changes and modifications may be made thereto without departing from the spirit of the invention, and it is intended to include all such changes and modifications as fall within the true scope of the invention.

## Claims

1. A laminate structure comprising:
an elastic fiber comprising polyurethane and 0.01% to 30% by weight of at least one additive selected from polystyrene;
wherein said elastic fiber is adhered to one or more layers of a substrate selected from the group consisting of fabric, nonwoven, film, and
combinations thereof.

2. The laminate structure of claim 1, wherein said elastic fiber is adhered by an adhesive, ultrasonic bonding, thermal bonding or combinations thereof.

3. The laminate structure of claim 1, wherein said elastic fiber is solution-spun.

4. The laminate structure of claim 1, wherein said elastic fiber is a sheath-core bicomponent fiber and said additive is present only in said sheath; optionally wherein said sheath includes said additive in an amount of 0.5% to 70% by weight of said sheath.

5. The laminate structure of claim 1, wherein said elastic fiber further comprises a topical finish comprising mineral oil, silicon oil, or combinations thereof in an amount of 0.1 % to 2% by weight of the fiber; optionally wherein said topical finish has a viscosity of about 5 centistokes to 150 centistokes.

6. The laminate structure of claim 1, wherein said elastic fiber further comprises an additional additive selected from magnesium stearate, organic stearates, silicon oil, mineral oil, and combinations thereof.

7. A method for preparing a laminate structure comprising:
providing an elastic fiber comprising polyurethane and 0.01% to 30% by weight of at least one additive selected from polystyrene;
wherein said elastic fiber is adhered to one or more layers of a substrate selected from the group consisting of fabric, nonwoven, film, and combinations thereof.

8. The method of claim 7, wherein said elastic fiber is adhered by ultrasonic bonding, an adhesive, thermal bonding, and combinations thereof.

9. The method of claim 7, wherein said laminate article is included in a disposable hygiene article.

## Patentansprüche

1. Laminatstruktur, umfassend:
eine elastische Faser, umfassend Polyurethan und 0,01 Gew.-% bis 30 Gew.-% an wenigstens einem Zusatzstoff ausgewählt aus Polystyrol;
wobei die elastische Faser an einer oder mehreren Schichten eines Substrats ausgewählt aus der Gruppe bestehend aus Gewebe, Vlies, Film und Kombinationen davon haftet.

2. Laminatstruktur gemäß Anspruch 1, wobei die elastische Faser durch einen Klebstoff, Ultraschallbindung, Wärmebindung oder Kombinationen davon haftet.

3. Laminatstruktur gemäß Anspruch 1, wobei die elastische Faser lösungsgesponnen ist.

4. Laminatstruktur gemäß Anspruch 1, wobei die elastische Faser eine Hülle-Kern-Bikomponentenfaser ist und der Zusatzstoff nur in der Hülle vorhanden ist; gegebenenfalls wobei die Hülle den Zusatzstoff in einer Menge von 0,5 Gew.-% bis 70 Gew.-% der Hülle enthält.

5. Laminatstruktur gemäß Anspruch 1, wobei die elastische Faser ferner eine äußerliche Endbehandlung umfassend Mineralöl, Siliconöl oder Kombinationen davon in einer Menge von 0,1 Gew.-% bis 2 Gew.-% der Faser umfasst; gegebenenfalls wobei die äußerliche Endbehandlung eine Viskosität von etwa 5 Zentistokes bis 150 Zentistokes aufweist.

6. Laminatstruktur gemäß Anspruch 1, wobei die elastische Faser ferner einen zusätzlichen Zusatzstoff ausgewählt aus Magnesiumstearat, organischen Stearaten, Siliconöl, Mineralöl und Kombinationen davon umfasst.

7. Verfahren zur Herstellung einer Laminatstruktur, umfassend:
Bereitstellen einer elastischen Faser, die Polyurethan und 0,01 Gew.-% bis 30 Gew.-% an wenigstens einem Zusatzstoff ausgewählt aus Polystyrol umfasst;
wobei die elastische Faser an einer oder mehreren Schichten eines Substrats ausgewählt aus der Gruppe bestehend aus Gewebe, Vlies, Film und Kombinationen davon haftet.

8. Verfahren gemäß Anspruch 7, wobei die elastische Faser durch Ultraschallbindung, einen Klebstoff, Wärmebindung oder Kombinationen davon haftet.

9. Verfahren gemäß Anspruch 7, wobei der Laminatgegenstand in einem Einweg-Hygienegegenstand enthalten ist.

## Revendications

1. Structure stratifiée comprenant :
une fibre élastique comprenant du polyuréthane et 0,01 % à 30 % en poids d'au moins un additif choisi dans le groupe constitué d'un polystyrène ;
dans laquelle ladite fibre élastique adhère à une ou plusieurs couches d'un substrat choisi dans le groupe constitué d'un tissu, un non-tissé, un film, et des combinaisons de ceux-ci.

2. Structure stratifiée selon la revendication 1, dans laquelle ladite fibre élastique adhère au moyen d'un adhésif, une liaison ultrasonore, une liaison thermique ou des combinaisons de ceux-ci.

3. Structure stratifiée selon la revendication 1, dans laquelle ladite fibre élastique est filée en solution.

4. Structure stratifiée selon la revendication 1, dans laquelle ladite fibre élastique est une fibre à deux composants gaine-noyau et ledit additif est présent uniquement dans ladite gaine ; facultativement dans laquelle ladite gaine comprend ledit additif en une quantité de 0,5 % à 70 % en poids de ladite gaine.

5. Structure stratifiée selon la revendication 1, dans laquelle ladite fibre élastique comprend en outre une finition topique comprenant une huile minérale, une huile silicone, ou des combinaisons de celles-ci en une quantité de 0,1 % à 2 % en poids de la fibre ; facultativement dans laquelle ladite finition topique a une viscosité d'environ 5 centistokes à 150 centistokes.

6. Structure stratifiée selon la revendication 1, dans lequel ladite fibre élastique comprend en outre un additif supplémentaire choisi parmi le stéarate de magnésium, des stéarates organiques, une huile de silicone, une huile minérale, et des combinaisons de ceux-ci.

7. Procédé de préparation d'une structure stratifiée comprenant :
la fourniture d'une fibre élastique comprenant du polyuréthane et 0,01 % à 30 % en poids d'au moins un additif choisi dans le groupe constitué d'un polystyrène ;
dans lequel ladite fibre élastique adhère à une ou plusieurs couches d'un substrat choisi dans le groupe constitué d'un tissu, un non-tissé, un film, et des combinaisons de ceux-ci.

8. Procédé selon la revendication 7, dans lequel ladite fibre élastique adhère au moyen d'une liaison ultrasonore, un adhésif, une liaison thermique, et des combinaisons de ceux-ci.

9. Procédé selon la revendication 7, dans lequel ledit article stratifié est inclus dans un article d'hygiène jetable.
